# EUROPÄISCHE PATENTANMELDUNG

(11) **EP 0 654 246 A1**
(43) Veröffentlichungstag der Anmeldung: **24.05.1995**
(21) Anmeldenummer: 94117682.8
(22) Anmeldetag: 09.11.1994
(51) Int. Cl.: A61B 17/28

(54) **Medizinisches Instrument mit einem zentralen Arbeitsstab und mit einem Handgriff**

(30) Priorität: 18.11.1993 DE 9317664 U
(71) Anmelder: DELMA ELEKTRO-UND MEDIZINISCHE APPARATEBAU GESELLSCHAFT mbH, D-78532 Tuttlingen (DE)
(72) Erfinder: Fritzsch, Gernod, Dr. Ing., D-78532 Tuttlingen (DE); Hermle, Michael, Dipl.-Ing., D-78532 Tuttlingen (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(57) **Zusammenfassung**

Ein medizinisches Hochfrequenz-Chirurgieinstrument weist einen zentralen Arbeitsstab auf, an dessen vorderem Ende ein oder mehrere Arbeitswerkzeuge 35 vorgesehen sind. Am hinteren Ende des Arbeitsstabes 1 ist ein Handgriff 2 vorgesehen. Der Arbeitsstab 1 ist relativ zum Handgriff 2 um seine Längsachse verdrehbar und in bestimmten Drehpositionen feststellbar. Zwischen Handgriff 2 und Arbeitsstab 1 sind radial innerhalb von dessen Außenumfang Rastmittel 30 vorhanden, die in vorbestimmten Verdrehstellungen des Arbeitsstabes 1 relativ zum Handgriff 2 automatisch einrasten und einer im Sinne einer weiteren Verdrehung des Arbeitsstabes 1 relativ zum Handgriff 2 ausgeübten Verdrehkraft eine vorbestimmte Haltekraft entgegensetzen.

## Beschreibung

Die Erfindung betrifft ein medizinisches Instrument, insbesondere Hochfrequenzchirurgieinstrument nach dem Oberbegriff des Schutzanspruches 1.

Bei derartigen medizinischen Instrumenten, insbesondere Hochfrequenz-Chirurgieinstrumenten, besteht oftmals die Notwendigkeit, das oder die am vorderen Ende des zentralen Arbeitsstabes angeordneten Arbeitswerkzeuge relativ zu der Arbeitsstelle um die Längsachse des Instruments zu drehen, um den Arbeitsvorgang optimal durchführen zu können. Es ist dabei nachteilig, wenn der Handgriff zur Verdrehung des Arbeitswerkzeuges mitverdreht werden muß, insbesondere wenn mit dem Handgriff zusätzlich andere Tätigkeiten ausgeführt werden müssen, wie beispielsweise das Betätigen eines zangenartigen Arbeitswerkzeuges.

Es ist daher bekannt, das Arbeitswerkzeug zusammen mit dem Arbeitsstab relativ zum Handgriff verdrehbar auszugestalten (DE-Gbm 91 14 306). Zur Arretierung in einer bestimmten Drehstellung müssen eine das gesamte Instrument umhüllende Hülse und ein damit zusammenwirkender Flansch vorgesehen sein, was sehr aufwendig ist.

Erfindungsgemäß wird vorgeschlagen, bei einem medizinischen Instrument, insbesondere Hochfrequenz-Chirurgieinstrument, nach dem Oberbegriff des Schutzanspruches 1 zwischen Handgriff und Arbeitsstab radial innerhalb desen Außenumfanges und insbesondere nur auf einer Seite der Mittelachse wirkende Rastmittel vorzusehen, die in vorbestimmten Verdrehstellungen des Arbeitsstabes relativ zum Handgriff automatisch einrasten und einer im Sinne einer weiteren Verdrehung des Arbeitsstabes relativ zum Handgriff ausgeübten Verdrehkraft eine vorbestimmte Haltekraft entgegensetzen, bei deren Überschreitung durch die Verdrehkraft die Rastmittel automatisch wieder auslösen und eine ungehinderte Weiterverdrehung ermöglichen.

Durch diese Merkmale wird eine einfache Handhabbarkeit auf besonders vorteilhafte Weise mit einer sicheren Funktion verbunden. Durch das automatische Ein- und Ausrasten der Rastmittel wird eine sonst erforderliche Betätigungsvorrichtung vermieden. Hierdurch wird nicht nur der Aufbau des erfindungsgemäßen Instruments vereinfacht, sondern auch der Bediener des Instruments entlastet. Die Anzahl der Verdrehstellungen des Arbeitswerkzeuges relativ zum Handgriff kann entsprechend den Anforderungen des jeweiligen Instrumentes gewählt werden. Da die Rastmittel nur auf einer Seite der Mittelachse wirken, ist die Anordnung kompakt und wenig platzaufwendig.

Nach einer besonderen Ausgestaltung der Erfindung ist ein Lochkranz mit einem der beiden gegeneinander verdrehbaren Teile drehschlüssig verbunden und in dem anderen verdrehbaren Teil oder einem mit diesem drehschlüssig verbundenen Teil mindestens ein in Richtung auf den Lochkranz axial verschieblicher Stift gelagert, dessen dem Lochkranz zugewandtes Ende sphärisch abgerundet ist, um ein teilweises Eintauchen dieses Endes des Stiftes in die Löcher des Lochkranzes zu ermöglichen, wobei der Stift kraftschlüssig mit seinem sphärischen Ende gegen den Lochkranz gepreßt wird. Diese Ausgestaltung ist vorteilhafterweise konstruktiv besonders einfach und dadurch auch funktionssicher. Die axial verschieblichen Stifte rasten mit ihrem dem Lochkranz zugewandten Ende in die Löcher des Lochkranzes ein. Durch die sphärische Ausbildung dieses dem Lochkranz zugewandten Endes wird ein automatisches Auslösen der Verrastung ermöglicht. Das sphärische Ende des Stiftes ruht in einem der Löcher des Lochkranzes in Art eines Kugelschnäppers, so daß bei einem weiteren Verdrehen von Handgriff und Arbeitswerkzeug gegeneinander von dem Rand des jeweiligen Loches des Lochkranzes eine Kraft auf das sphärische Ende des Stiftes ausgeübt wird, die der Kraft der Rückstelleinrichtung entgegenwirkt. Überschreitet diese Kraft die Kraft der Rückstelleinrichtung, so wird der Stift aus dem Loch ausgehoben. Nun ist eine weitere Verdrehung des Arbeitswerkzeuges relativ zum Handgriff ohne Gegenkraft möglich, bis das sphärische Ende des Stiftes in das nächste Loch des Lochkranzes einrastet.

Durch die Anzahl der Löcher des Lochkranzes und deren Anordnung können die verschiedenen gewünschten Raststellungen bestimmt werden. Es können auch mehrere Stifte vorgesehen sein, beispielsweise um eine höhere Rastkraft zu bewirken, oder um diese räumlich gleichmäßig zu verteilen.

Nach einer weiteren Ausgestaltung der Erfindung weist der Handgriff einen rohrförmigen Abschnitt auf zur Aufnahme und axialen Führung des hinteren Endes des Arbeitsstabes. Weiterhin ist der Arbeitsstab in diesem Abschnitt auslösbar arretierbar. Diese Ausgestaltung hat den Vorteil, daß der Arbeitsstab, beispielsweise zu Reinigungszwecken, von dem Handgriff gelöst werden kann.

Nach einer weiteren Ausgestaltung der Erfindung ist in den Außenumfang des hinteren Endes des Arbeitsstabes eine Ringnut eingearbeitet, in welche ein Kupplungsriegel einrastbar ist. Der Kupplungsriegel weist hierzu eine Durchtrittsöffnung für den Arbeitsstab auf, deren seitlicher Rand mit der Ringnut des Arbeitsstabes zusammenwirkt, um eine Axialbewegung des Arbeitsstabes zu verhindern. Der Kupplungsriegel ist zwischen zwei Stellungen quer zur Längsachse des Arbeitsstabes verschieblich geführt, wobei eine Rückstelleinrichtung den Kupplungsriegel in der die Axialbewegung des Arbeitsstabes verhindernden Stellung hält. Diese Ausgestaltung ermöglicht vorteilhafterweise eine sichere auslösbare Arretierung des Arbeitsstabes in dem rohrförmigen Abschnitt des Handgriffs. Durch Einbringen einer der Rückstelleinrichtung entgegenwirkenden Kraft wird der Kupplungsriegel in seine den Durchtritt des Arbeitsstabes freigebende Stellung bewegt zum Einführen bzw. Herausziehen des Arbeitsstabes. Nach Ende des Krafteintrags wird durch die Rückstelleinrichtung der Kupplungsriegel automatisch in seine den Arbeitsstab arretierende Stellung verschoben.

Nach einer weiteren Ausgestaltung der Erfindung ist um den Arbeitsstab ein Schutzrohr, bevorzugt koaxial, angeordnet, welches mit dem Arbeitsstab bezüglich einer Drehung um ihre Längsachsen drehschlüssig verbunden ist. Insbesondere bei Hochfreguenz-Chirurgieinstrumenten wird um den Arbeitsstab ein Schutzrohr benötigt, welches den Arbeitsstab und die daran vorhandenen Einrichtungen einerseits vor Beschädigungen schützt und andererseits einen Kontakt mit stromführenden Teilen verhindert. Durch den Drehschluß Zwischen Schutzrohr und Arbeitsstab wird eine Verdrehung des Arbeitsstabes und der daran vorhandenen Arbeitswerkzeuge über das diesen umgebende Schutzrohr ermöglicht. Die Rastmittel können dabei entweder am Arbeitsstab oder am Schutzrohr und andererseits am Handgriff angeordnet sein.

Nach einer weiteren Ausgestaltung der Erfindung weist der Arbeitsstab mindestens in einem Teilbereich drei-, vier- oder mehreckigen Querschnitt auf und das Schutzrohr mindestens in einem diesem Teilbereich zugeordneten Abschnitt einen entsprechenden Innenquerschnitt, wobei der freie Innenquerschnitt des Schutzrohres etwas größer ist als der Umfang des Arbeitsstabes, um eine freie Axialbewegung des Arbeitsstabes relativ zum Schutzrohr zu ermöglichen. Durch diese Ausgestaltung wird auf geeignete Weise ein Drehschluß zwischen Schutzrohr und Arbeitsstab bewirkt und gleichzeitig eine leichtgängige Axialbewegung des Arbeitsstabes ermöglicht. Dies ist insbesondere bei solchen Hochfrequenz-Chirurgieinstrumenten vorteilhaft, die als Arbeitswerkzeug ein innerhalb des Arbeitsstabes gelagertes zangenförmiges Werkzeug aufweisen, dessen Zangenbewegung durch Axialbewegung des Arbeitsstabes relativ zum Schutzrohr bewirkt wird.

Nach einer weiteren Ausgestaltung der Erfindung ist in das Schutzrohr in einem dem Teilbereich eckigen Querschnitts des Arbeitsstabes zugeordneten Abschnitt eine Lamellenbuchse eingepreßt, deren freier Innenquerschnitt eine dem Querschnitt des Arbeitsstabes entsprechende Form aufweist. Durch diese Ausgestaltung kann das Schutzrohr vorteilhafterweise beispielsweise kreiszylinderförmig ausgebildet werden, während der Drehschluß durch die eingepreßte Lamellenbuchse bewirkt wird. Hierdurch wird der Konstruktionsaufwand des Schutzrohres verringert.

Nach einer weiteren Ausgestaltung der Erfindung ist das Schutzrohr lösbar mit dem Handgriff verbunden. Diese Ausgestaltung hat den Vorteil, daß das erfindungsgemäße Instrument, beispielsweise zu Reinigungszwecken, zerlegbar ist. Das Schutzrohr ist hierbei bevorzugt über eine Schnappverbindung am Handgriff arretierbar und durch Gegenzug wieder lösbar. Das Verlegen und Zusammensetzen des erfindungsgemäßen Instruments ist dadurch besonders einfach.

Nach einer weiteren Ausgestaltung der Erfindung sind Mittel vorhanden, um bei entferntem Lochkranz die Axialbewegung der Stifte zu begrenzen. Diese Ausgestaltung hat den Vorteil, daß bei zerlegtem Instrument die von der Rückstelleinrichtung belasteten Stifte nicht aus ihrer Lagerung herausfallen.

Ein Ausführungsbeispiel der Erfindung ist in der Zeichnung dargestellt und wird nachfolgend beschrieben. Es zeigen:
- Fig. 1: einen Längsschnitt durch ein erfindungsgemäßes Hochfrequenz-Chirurgieinstrument,
- Fig. 2: einen Schnitt gemäß Linie II-II in Fig. 1 in gegenüber Fig. 1 leicht vergrößerter Darstellung,
- Fig. 3: eine schematische Schnittansicht nach Linie III-III in Fig. 2 und die
- Fig.4,5: Schnittansichten entsprechend III-III in Fig. 2 zweier weiterer vorteilhafter Ausführungsformen der Rastflächen und Rastgegenflächen der erfindungsgemäßen Rastmittel.

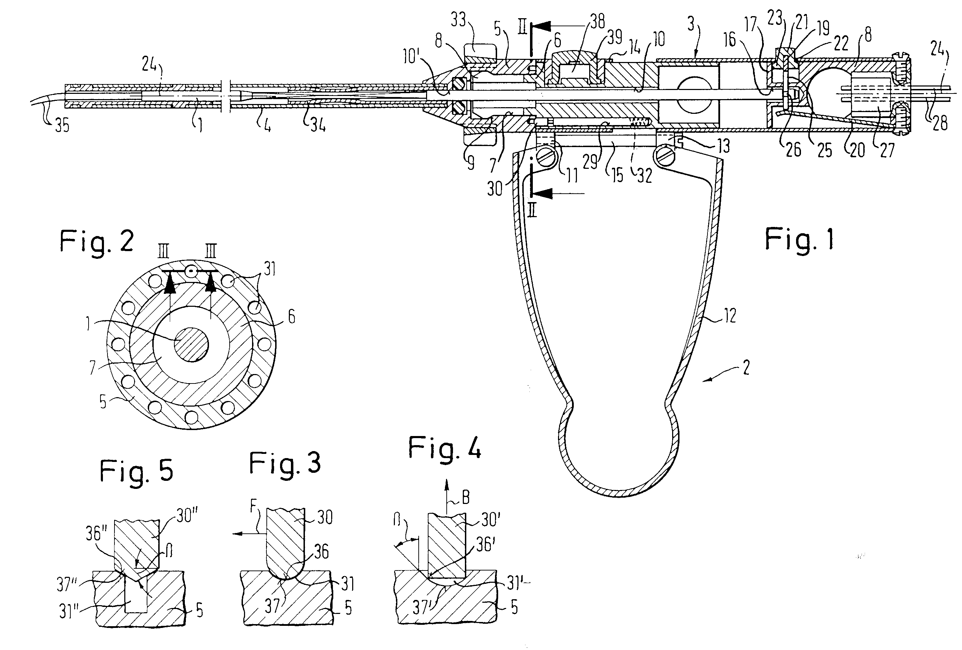

Das in Fig. 1 dargestellte Hochfrequenz-Chirurgieinstrument weist einen als Elektrodenrohr ausgebildeten zentralen Arbeitsstab 1 auf, dessen hinteres Ende in einem rohrförmigen Abschnitt 3 eines Handgriffes 2 axial verschiebbar gelagert ist und dessen vorderes Ende von einem schützenden koaxialen Führungsrohr 4 umgeben ist und zwei federnd voneinander weg vorgespannte Arbeitselektroden 35 trägt. Das hintere Ende des Führungsrohres 4 ist in ein Verbindungsstück 5 axial und in Umfangsrichtung fest eingesetzt, welches mit einem Anschlußstück 6, welches im Handgriff 2 vorhanden ist, eine Schnappverbindung bildet.

Das Verbindungsstück 5 weist hierzu eine zentrale, in Richtung auf das vordere Ende des Elektrodenrohres 1 sich erweiternde Stufenbohrung 7 kreisförmigen Querschnitts auf. Am Ansatzstück 6 sind parallel zum Elektrodenrohr 1 sich in Richtung auf dessen vorderes Ende erstreckende Federzungen 8 angeordnet, deren radial außenliegende Seite entsprechend der Stufenbohrung 7 gestuft ausgebildet ist, um die Stufe 9 der Stufenbohrung 7 federnd zu hintergreifen.

Das Verbindungsstück 5 ist auf der Schnappverbindung mit dem Anschlußstück 6 um die Längsachse 24 des Instrumentes drehbar, wobei zur Erleichterung der Drehbewegung am Verbindungsstück 5 radial nach außen weisende Flügel 33 vorhanden sind. In dem mit dem Verbindungsstück 5 verbundenen Führungsrohr 4 ist eine Lamellenbuchse 34 drehfest eingepreßt, deren Lamellen eine Drehung des Führungsrohres 4 über den hierfür in diesem Abschnitt vierkantförmig ausgebildeten Außenumfang des Elektrodenrohres 1 auf dieses übertragen, jedoch eine freie Axialbewegung des Elektrodenrohres 1 relativ zum Führungsrohr 4 ermöglichen.

Das Verbindungsstück 5 und das Anschlußstück 6 weisen je eine zentrale Bohrung 10, 10' auf, durch welche das Elektrodenrohr 1 geführt ist. Das Anschlußstück 6 ist über ein Ansatzstück 11 mit einem Griffteil des Handgriffs 2 verbunden, welches als Zweiarmiger Federbügel 12 ausgebildet ist, dessen einer Arm mit dem Ansatzstück 11 des Anschlußstückes 6 verbunden ist und dessen anderer Arm über ein Ansatzstück 13 mit dem rohrförmigen Abschnitt 3 des Handgriffs 2 verbunden ist. Der rohrförmige Abschnitt 3 des Handgriffs 2 umfaßt das hintere Ende des Anschlußstücks 6 und ist auf diesem axial verschieblich gleitend geführt.

Durch Aufeinanderzubewegen der Arme des Bügel 12 wird der rohrförmige Abschnitt 3 relativ zum Anschlußstück 6 axial nach vorne geschoben und dadurch das Elektrodenrohr 1 aus dem Führungsrohr 4 herausbewegt, so daß die am vorderen Ende des Elektrodenrohres 1 vorhandenen Arbeitselektroden 35 sich öffnen.

Das Anschlußstück 6 ist von einem Abdeckrohr 14 umgeben, welches einen Anschlag für die Axialbewegung des rohrförmigen Abschnittes 3 nach vorn bildet. In entgegengesetzter Richtung ist die Axialbewegung des rohrförmigen Abschnitts des Handgriffs 2 durch eine Anschlagschraube 15 begrenzt, welche durch eine Ausnehmung im Ansatzstück 13 geführt und mit ihrer Schaftspitze am Ansatzstück 11 verankert ist.

Der hintere Endbereich des Elektrodenrohres 1 erstreckt sich durch eine Bohrung 16 in einem an eine Ringwand 17 sich anschließenden Einsatz 18 im rohrförmigen Abschnitt 3 bis zu einem querverschiebbar in diesem angeordneten Kupplungsriegel 19, der von einer Riegelfeder 20 diametral gegen einen Druckknopf 21 vorgespannt ist, welcher sich durch eine Bohrung 22 in der Wand des rohrförmigen Abschnittes 3 und dem Einsatz 18 radial nach außen erstreckt und dessen Bewegung radial nach außen durch eine im Inneren des rohrförmigen Abschnittes 3 liegende radiale Erweiterung 23 begrenzt ist.

Der Kupplungsriegel 19 erstreckt sich durch radiale Ausnehmungen im Einsatz 18 und ist durch diese derart in Richtung quer zur Längsachse 24 des Instrumentes geführt, daß er axiale Belastungen aufnehmen kann. Der Kupplungsriegel 19 weist eine zentrale Öffnung 25 auf, die den hinteren Endbereich des Elektrodenrohres 1 umgibt und deren untere Berandung durch die Riegelfeder 20 in eine im Elektrodenrohr 1 vorgesehene Ringnut 26 eingreift. Da die Dicke des plattenförmig ausgebildeten Kupplungsriegels 19 und die axiale Erstreckung der Ringnut 26 praktisch gleich sind, so daß der Kupplungsriegel 19 radial in die Ringnut 26 eintreten kann, liegt eine axiale Verankerung zwischen dem Kupplungsriegel 19 und dem Elektrodenrohr 1 vor, welches eine axiale Verschiebung des Elektrodenrohrs 1 innerhalb des rohrförmigen Abschnitts 3 verhindert, eine Drehung des Elektrodenrohres 1 relativ zum Handgriff 2 um die Achse 24 jedoch ermöglicht.

Durch Drücken auf den Druckknopf 21 kann die Verankerung zwischen dem Kupplungsriegel 19 und dem hinteren Endbereich des Elektrodenrohres 1 gelöst werden, wodurch das Elektrodenrohr 1 nach vorne aus dem Führungsrohr 4 herausgezogen werden kann.

Am hinteren Ende des rohrförmigen Abschnitts 3 ist ein Stecker 27 eingesetzt mit zwei Zuführungsleitungen 28 für die Hochfrequenzspannung, die über nicht dargestellte Kontaktfedern mit dem Elektrodenrohr 1 in Verbindung stehen.

Im Anschlußstück 6 des Handgriffs 2 ist eine parallel zur Achse 24 des Instrumentes sich erstreckende Bohrung 29 vorgesehen, in der ein Raststift 30 axial verschiebbar geführt ist. In der dem Anschlußstück 6 zugewandten Stirnseite des Verbindungsstücks 5 ist gemäß Fig. 1 bis 3 ein Lochkranz mit Rastlöchern 31 vorgesehen, gegen die das teilsphärisch ausgebildete Ende des Raststiftes 30 durch eine Druckfeder 32 gepreßt wird. Die Druckfeder 32 ist im Anschlußstück 6 verankert, und es ist außerdem eine Halteeinrichtung vorgesehen, die ein Herausfallen des Raststiftes 30 aus der Bohrung 29 verhindert. Wesentlich ist, daß die Rastfläche 36 des Raststiftes 30 und/oder die Rastgegenflächen 37 der Rastlöcher 31 so flach sind, daß bei Verdrehung des den Raststift 30 tragenden Handgriffs 2 in Richtung des Pfeiles F in Fig. 3 der Raststift 30 unter axialer Verschiebung in Richtung der Druckfeder 32 aus dem Rastloch 31 austreten kann, ohne daß ein besonderes Betätigungsmittel für den Raststift 30 vorgesehen ist.

Die Arbeitsweise des beschriebenen Hochfrequenz-Chirurgieinstrumentes ist wie folgt:
Durch Schließen eines Schalters kann über die Zuführungsleitung 28 eine Hochfrequenzspannung angelegt werden. Durch Aufeinanderzubewegen der beiden Arme des Bügels 12 wird das Führungsrohr 4 relativ zum Elektrodenrohr 1 nach hinten verschoben, wodurch die beiden Elektroden 35 aus dem Führungsrohr 4 herausbewegt werden und sich die beiden Elektroden 35 aufgrund der federnden Vorspannung voneinander wegspreizen. Beim anschließenden Wiedervorschieben des Führungsrohres 4 aufgrund der Federkraft des Bügels 12 werden die beiden Elektroden 35 wieder aufeinander zubewegt, so daß eine zangenartige Bewegung der beiden Arbeitselektroden 35 relativ zueinander hervorgerufen werden kann.

Während eines solchen Arbeitsvorganges ist der Raststift 30 in eines der Rastlöcher 31 des Lochkranzes eingerastet, so daß zwischen den Arbeitselektroden 35 und dem Handgriff 2 eine definierte feste Winkelbeziehung besteht. Um nun die Arbeitselektroden 35 in eine Winkelposition relativ zum Handgriff 2 zu bringen, wird bei festgehaltenem Handgriff 2 das Verbindungsstück 5 über die Flügel 33 um die Achse 24 verdreht. Die hierbei ausgeübte Drehkraft wirkt über den Rand des Rastloches 31, in welches der Raststift 30 eingerastet ist, auf die teilsphärische Rastfläche 36 des Raststiftes 30, um diesen entgegen der Kraft der Druckfeder 32 axial zu verschieben. Sobald die Verdrehkraft die Rastkraft übersteigt, beginnt das Verbindungsstück 5 sich relativ zum Handgriff zu verdrehen, wobei der Raststift 30 in die Bohrung 29 zurückgeschoben wird. Die teilsphärische Rastfläche 36 des Raststiftes 30 wird dabei aus dem Rastloch 31 ausgehoben und bewegt sich anschließend auf der den Lochkranz aufweisenden Stirnfläche des Verbindungsstücks 5 in Umfangsrichtung gleitend, bis das nächste Rastloch 31 mit dem Raststift 30 fluchtet und dieser darin einrastet.

Auf diese Weise kann das erfindungsgemäße Hochfrequenz-Chirurgieinstrument mit einer von der Anzahl der Rastlöcher 31 abhängigen Zahl unterschiedlicher Winkelpositionen der Arbeitselektroden 35 eingesetzt werden, ohne daß der Handgriff 2 ebenfalls mitverdreht werden muß, so daß die Betätigung des Bügels 12 des Handgriffs 2 in einer ergonomisch und den Platzverhältnissen entsprechend günstigen Winkellage erfolgen kann.

Zum Zerlegen des Instruments wird der Druckknopf 21 gedrückt, wodurch der Kupplungsriegel 19 außer Eingriff mit dem Elektrodenrohr 1 kommt. Dieses kann dann nach vorne vollständig aus dem Führungsrohr 4 herausgezogen werden. Das Führungsrohr 4 kann von dem Handgriff 2 dadurch getrennt werden, daß die Schnappverbindung durch Einbringen einer axialen Abzugskraft gelöst wird.

Nach Fig. 4 kann die Rastfläche 36' des Raststiftes 30' auch als Umfangskante am flachen vorderen Ende ausgebildet sein, welche an einer flachen teilsphärischen oder konischen Rastgegenfläche 37' des Rastloches 31' anliegt. Der Winkel β zwischen der Bewegungsrichtung B des Raststiftes 30' und der aus dem Rastloch 31' herausführenden Rastgegenfläche, wie er durch Fig. 4 definiert ist, muß so flach sein, daß bei Verdrehung des Verbindungsstückes 5 relativ zum Handgriff 2 der Raststift 30' zwanglos aus dem Rastloch 31' herausgeholt wird.

Fig. 5 zeigt, daß die flach konische oder teilsphärische Rastfläche 36'' auch am freien Ende des Raststiftes 30'' vorgesehen sein kann und mit einer als Eingangskante eines zylindrischen Rastloches 31'' ausgebildete Rastgegenfläche 37'' ausgebildet sein kann. Auch hier muß der Winkel β die vorstehend erwähnte Bedingung erfüllen.

### Liste der Bezugszeichen

- 1: Elektrodenrohr
- 2: Handgriff
- 3: rohrförmiger Abschnitt
- 4: Führungsrohr
- 5: Verbindungsstück
- 6: Anschlußstück
- 7: Stufenbohrung
- 8: Federzunge
- 9: Stufe
- 10, 10': zentrale Bohrung
- 11: Ansatzstück
- 12: Bügel
- 13: Ansatzstück
- 14: Abdeckrohr
- 15: Anschlagschraube
- 16: Bohrung
- 17: Ringwand
- 18: Einsatz
- 19: Kupplungsriegel
- 20: Riegelfeder
- 21: Druckknopf
- 22: Bohrung
- 23: radiale Erweiterung
- 24: Längsachse
- 25: Öffnung
- 26: Ringnut
- 27: Stecker
- 28: Zuführungsleitung
- 29: Bohrung
- 30,30', 30'': Raststift
- 31,31', 31'': Rastloch
- 32: Federdruckelement
- 33: Flügel
- 34: Lamellenbuchse
- 35: Arbeitselektroden
- 36,36', 36'': Rastflächen
- 37,37', 37'': Rastgegenflächen

## Patentansprüche

1. Medizinisches Instrument, insbesondere Hochfrequenz-Chirurgieinstrument, mit einem zentralen Arbeitsstab (1), insbesondere Elektrodenrohr, an dessen vorderen Ende ein oder mehrere Arbeitswerkzeuge (35), insbesondere Arbeitselektroden, und vorzugsweise an dessen hinterem Ende Anschlüsse zur Versorgung der Arbeitswerkzeuge, insbesondere ein bzw. mehrere Hochfrequenzspannungsanschlüsse (27, 28), vorgesehen sind, und mit einem am hinteren Ende des Arbeitsstabes (1) angeordneten Handgriff (2), wobei der Arbeitsstab (1) relativ zum Handgriff (2) um seine Längsachse verdrehbar und in bestimmten Drehpositionen feststellbar ist,
dadurch **gekennzeichnet,**
daß zwischen Handgriff (2) und Arbeitsstab (1) radial innerhalb dessen Außenumfanges und insbesondere nur auf einer Seite der Mittelachse wirkende Rastmittel (30, 30', 30''; 31, 31', 31'') vorhanden sind, die in vorbestimmten Verdrehstellungen des Arbeitsstabes (1) relativ zum Handgriff (2) automatisch einrasten und einer im Sinne einer weiteren Verdrehung des Arbeitsstabes (1) relativ zum Handgriff (2) ausgeübten Verdrehkraft eine vorbestimmte Haltekraft entgegensetzen, bei deren Überschreitung durch die Verdrehkraft die Rastmittel (30, 30', 30''; 31, 31', 31'') automatisch wieder auslösen und eine ungehinderte Weiterverdrehung ermöglichen.

2. Medizinisches Instrument nach Anspruch 1,
dadurch **gekennzeichnet,**
daß eines (30, 30', 30'') der Rastmittel auf das andere (31, 31', 31'') zu federnd vorgespannt ist.

3. Medizinisches Instrument nach Anspruch 1,
dadurch **gekennzeichnet,**
daß ein Rastmittel als aus Rastlöchern (31, 31', 31'') bestehender Lochkranz oder ähnliche Kränze mit Vertiefungen ausgebildet und mit einem der beiden gegeneinander verdrehbaren Teile drehschlüssig verbunden ist, und daß in dem anderen verdrehbaren Teil oder einem mit diesem drehschlüssig verbundenen Teil mindestens ein in Richtung auf den Lochkranz axial verschieblicher Stift (30, 30', 30'') mit mäßiger Querkraft gelagert ist, wobei die miteinander in Eingriff tretenden Rastflächen (36, 36', 36'') des Raststiftes (30, 30', 30'') und/oder Rastgegenflächen (37, 37', 37'') der Rastlöcher (31, 31', 31'') derart schräg zur axialen Verschieberichtung des Raststiftes (30, 30', 30'') geneigt sind, daß bei seitlicher Bewegung des Raststiftes (30, 30', 30'') relativ zu einem Rastloch (31, 31', 31''), in das er eingerastet ist, eine axiale Rückstellkraft auf den Raststift (30, 30', 30'') ausgeübt wird, die ihn aus dem Rastloch (31, 31', 31'') heraushebt.

4. Medizinisches Instrument nach einem der vorhergehenden Ansprüche,
dadurch **gekennzeichnet,**
daß die Rastfläche (36) des Raststiftes (30) und die Rastgegenfläche (37) der Rastlöcher (31) flach teilsphärisch ausgebildet sind.

5. Medizinisches Instrument nach einem der Ansprüche 1 bis 3,
dadurch **gekennzeichnet,**
daß die Rastgegenfläche (37') der Rastlöcher (31') flach teilsphärisch oder sich konisch zum Raststift (30') hin erweiternd ausgebildet ist, wobei die Rastfläche (36') des Raststiftes (30') komplementär zum Rastloch oder mit an den Rastgegenflächen (37') gleitenden Kanten ausgebildet ist.

6. Medizinisches Instrument nach einem der Ansprüche 1 bis 3,
dadurch **gekennzeichnet,**
daß das Rastloch (31'') als zylindrische Bohrung ausgebildet ist, auf deren als Rastgegenfläche (37'') ausgebildeter Eintrittskante ein mit flach konischer oder teilsphärischer Rastfläche (36'') versehener Raststift (30'') anliegt.

7. Medizinisches Instrument nach einem der vorhergehenden Ansprüche,
dadurch **gekennzeichnet,**
daß der Handgriff (2) einen rohrförmigen Abschnitt (3) aufweist zur Aufnahme und axialen Führung des hinteren Endes des Arbeitsstabes (1), und daß der Arbeitsstab (1) in diesem Abschnitt (3) axial auslösbar, aber drehbar arretierbar ist.

8. Medizinisches Instrument nach Anspruch 3,
dadurch **gekennzeichnet,**
daß am hinteren Ende des Arbeitsstabes (1) eine Ringnut (26) in dessen Außenumfang vorhanden ist, in welche ein gegen die Kraft einer Rückstelleinrichtung (20) quer zur Längsachse (24) des Arbeitsstabes (1) verschiebbar in dem rohrförmigen Abschnitt (3) des Handgriffs (2) geführter Kupplungsriegel (19) einrastbar ist, welcher hierzu eine Durchtrittsöffnung (25) zur Durchführung des hinteren Endes des Arbeitsstabes (1) aufweist, deren seitlicher Rand mit der Ringnut (26) zur Arretierung des Arbeitsstabes (1) zusammenwirkt.

9. Medizinisches Instrument nach einem der vorhergehenden Ansprüche,
dadurch **gekennzeichnet,**
daß um den Arbeitstab (1) ein Schutzrohr (4) bevorzugt koaxial, angeordnet ist, welches mit dem Arbeitsstab (1) bezüglich einer Drehung um ihre Längsachsen drehschlüssig koppelbar ist, wobei insbesondere vorgesehen ist, daß der Arbeitsstab (1) mindestens in einem Teilbereich drei-, vier- oder mehreckigen Querschnitt aufweist und daß das Schutzrohr (4) mindestens in einem diesem Teilbereich zugeordneten Teilbereich einen entsprechenden Innenquerschnitt aufweist, wobei der freie Innequerschnitt des Schutzrohres (4) etwas größer ist als der Umfang des Arbeitstabs (1), um eine freie Axialbewegung des Arbeitsstabes (1) relativ zum Schutzrohr (4) zu gewährleisten, und daß bevorzugt in das Schutzrohr (4) in einem dem Teilbereich eckigem Querschnitt des Arbeitsstabes (1) zugeordneten Abschnitt eine Lammellenbuchse (34) eingepreßt ist, deren freier Innenquerschnitt eine dem Querschnitt des Arbeitsstabes (1) entsprechende Form aufweist.

10. Medizinisches Instrument nach Anspruch 9,
dadurch **gekennzeichnet,**
daß das Schutzrohr (4) lösbar mit dem Handgriff (2) verbunden ist, wobei die Ausbildung insbesondere so ist, daß das Schutzrohr (4) über eine Schnappverbindung am Handgriff 82) arretierbar und durch Druck auf den Schnappauslösemechanismus wieder lösbar ist, und/oder daß Mittel vorhanden sind, um bei entferntem Lochkranz die Axialbewegung der Stifte (30) zu begrenzen.
